# EUROPEAN PATENT SPECIFICATION

(11) **EP 4 418 984 B1**
(45) Date of publication and mention of the grant of the patent: **20.08.2025**
(21) Application number: 22793189.6
(22) Date of filing: 04.10.2022
(51) Int. Cl.: A61B 3/16

(54) **REBOUND TONOMETERS AND METHODS FOR USING REBOUND TONOMETERS**
REBOUND-TONOMETER UND VERFAHREN ZUR VERWENDUNG VON REBOUND-TONOMETERN
TONOMÈTRE À REBOND ET PROCÉDÉS D'UTILISATION DE TONOMÈTRE À REBOND

(30) Priority: 21.10.2021 FI 20216097
(43) Date of publication of application: 28.08.2024
(73) Proprietor: Icare Finland Oy, 01510 Vantaa (FI)
(72) Inventor: RAUDASOJA, Matti, 01340 Vantaa (FI); HONKANEN, Viktor, 02610 Espoo (FI); KANGAS, Lauri, 02210 Espoo (FI)
(74) Representative: Moosedog Oy
(86) International application number: PCT/FI2022/050660
(87) International publication number: WO 2023/067241

(56) References cited:
- EP-A1- 3 581 089
- WO-A1-2017/103330
- WO-A1-2019/055374
- WO-A1-2021/154769
- JP-A- 2021 037 119
- US-A- 6 093 147

## Description

### TECHNICAL FIELD

The present disclosure relates to rebound tonometers. Moreover, the present disclosure also relates to methods for using rebound tonometers for determining properties of objects.

### BACKGROUND

Over the past few decades, measurement devices have gained popularity in various disciplines such as medicine, engineering, and the like. Particularly, in the medicine discipline, the measurement devices are often employed to view objects (such as eyes of patients, body parts of patients, or similar), for example by medical professionals (such as optometrists, ophthalmologists, physicians, and the like), in order to measure parameters associated with the objects. As an example, a rebound tonometer may be employed to measure an intraocular pressure of an eye (namely, a fluid pressure inside the eye) of a patient by interacting with cornea of the eye to an indentation. Generally, when using such rebound tonometers, the rebound tonometers are arranged (namely, set) in front of the objects at a particular distance from the objects.

However, there exist very specific requirements of alignment of rebound tonometers with respect to the objects (in order to measure the parameters associated with the objects). In some cases, the existing rebound tonometers are arranged too far from the objects. In such cases, the parameters are inaccurately measured or are not measured at all. In other cases, when the existing rebound tonometers are arranged too close to the objects, part(s) of said tonometers could accidently harm the object while measuring the parameters. Resultantly, in both cases, unreliable and erroneous measurements are obtained.

A patent document EP3581089 discloses a method for determining intra-ocular pressure (IOP) is presented. The method calculates the first derivative of the probe's velocity at the point of zero velocity upon contact with the cornea, correlating it to the IOP for improved accuracy.

A patent document WO2019055374 discloses an improved rebound tonometer that incorporates a tilt sensor to enhance intra-ocular pressure (IOP) measurements. The sensor provides a tilt signal to the device's processor, which applies a tilt correction factor to the basic IOP value based on the direction and degree of tilt. This correction accounts for gravitational effects on the probe, allowing accurate measurements even at small tilt angles.

A patent document WO2017103330 discloses an apparatus for measuring intraocular pressure (IOP) that features a functional part with a tubular probe base housing a probe. The probe, partly made of magnetic material, contacts the eye's surface to determine IOP from its velocity changes. An induction coil accelerates the probe to a specific velocity. The apparatus includes systems for measuring the probe's velocity variations, processing and displaying data, and managing operations.

Some existing rebound tonometers comprise holders for contacting the surrounding portions of the object in a manner that it facilitates in arranging the rebound tonometers in front of the objects. The holders define a distance between the rebound tonometers and the objects. However, such holders arrange the rebound tonometers in front of the objects inaccurately and imprecisely. This is because said holders are mechanically set on the rebound tonometers, and are arranged manually with respect to the objects, thus being error-prone on account of such mechanical setup and manual intervention. As an example, the rebound tonometer may comprise a holder that is capable of making a contact with a forehead of a patient when measuring an intraocular pressure of an eye of the patient. Such a holder cannot compensate for different form factors of forehead geometries of different patients, and thus is unable to accurately align the rebound tonometer with the eye. Furthermore, the rebound tonometers with holders are heavy in terms of weight, and are expensive. Use of typical distance measurement solutions such as ultrasonic sensors, cameras, and similar, with existing rebound tonometers is not feasible since these solutions are expensive and hard to implement.

Therefore, in light of the foregoing discussion, there exists a need to overcome the aforementioned drawbacks associated with existing rebound tonometers.

### SUMMARY

The present disclosure seeks to provide a rebound tonometer. The present disclosure also seeks to provide a method for using a rebound tonometer for determining a property of an object. An aim of the present disclosure is to provide a solution that overcomes at least partially the problems encountered in prior art.

In one aspect, an embodiment of the present disclosure provides a rebound tonometer comprising:
a body having a proximal end and a distal end opposite to the proximal end, wherein the body has an opening at the proximal end;
a magnetic elongated probe having a first end and a second end opposite to the first end, wherein the magnetic elongated probe is arranged at least partially in the body in a manner that the first end protrudes outside of the body from the opening of the body, the first end is at a first distance from the opening, and the second end is inside the body, and wherein the magnetic elongated probe is arranged to be aligned with and movable along an axis of the rebound tonometer;
a measurement coil and a drive coil arranged inside the body and to partially surround the magnetic elongated probe; and a controller configured to:
   - detect a contact between an object and the first end, when the rebound tonometer is in use, by:
      - energizing the drive coil to move the magnetic elongated probe in respect to the body to have the first end at a second distance from the proximal end, the second distance being greater than the first distance;
      - measuring a first induced voltage in the measurement coil as a function of time during a first time period; and
      - comparing the measured first induced voltage as a function of time with a predetermined criterion, and if based on the comparison, the predetermined criterion is met, use it as an indication of a detected contact between the first end and the object; and
   - initiate a measurement cycle of the rebound tonometer when the contact is detected.

In another aspect, an embodiment of the present disclosure provides a method for using a rebound tonometer for determining a property of an object, wherein the rebound tonometer comprises a body, a magnetic elongated probe, a measurement coil and a drive coil, the method comprising:
- arranging the magnetic elongated probe at least partially in the body in a manner that a first end of the magnetic elongated probe protrudes outside of the body from an opening at a proximal end of the body, the first end is at a first distance from the opening, and a second end of the magnetic elongated probe is inside the body, wherein the magnetic elongated probe is arranged to be movable along an axis of the rebound tonometer;
- detecting a contact between the object and the first end by:
   - moving the magnetic elongated probe in respect to the body to have the first end at a second distance from the proximal end, the second distance being greater than the first distance;
   - moving the rebound tonometer in respect to the object during a first time period;
   - measuring a first induced voltage as a function of time during a first time period as the rebound tonometer is moved; and
   - comparing the measured first induced voltage as a function of time with a predetermined criterion, and if based on the comparison the predetermined criterion is met, using it as an indication of a detected contact between the first end and the object; and
- initiating a measurement cycle of the rebound tonometer when the contact is detected.

Embodiments of the present disclosure substantially eliminate or at least partially address the aforementioned problems in the prior art, and enable accurate and reliable alignment of a rebound tonometer with respect to an object during a measurement cycle for measuring a property of the object.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1 is an exemplary schematic diagram of a rebound tonometer, in accordance with an embodiment of the present disclosure;
FIGs. 2A, 2B and 2C are exemplary schematic diagrams of usage states of a rebound tonometer, in accordance with various embodiments of the present disclosure;
FIG. 3 is a block diagram of a rebound tonometer, in accordance with an embodiment of the present disclosure;
FIG. 4A illustrates a first waveform of a first induced voltage in a measurement coil as a function of time during a first time period, while FIG. 4B illustrates a second waveform of a second voltage that is induced in the measurement coil as a function of time during a second time period, in accordance with an embodiment of the present disclosure; and
FIG. 5 is a flowchart depicting steps of a method for using a rebound tonometer for determining a property of an object, in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In one aspect, an embodiment of the present disclosure provides rebound tonometer comprising:
a body having a proximal end and a distal end opposite to the proximal end, wherein the body has an opening at the proximal end;
a magnetic elongated probe having a first end and a second end opposite to the first end, wherein the magnetic elongated probe is arranged at least partially in the body in a manner that the first end protrudes outside of the body from the opening of the body, the first end is at a first distance from the opening, and the second end is inside the body, and wherein the magnetic elongated probe is arranged to be aligned with and movable along an axis of the rebound tonometer;
a measurement coil and a drive coil arranged inside the body and to partially surround the magnetic elongated probe; and
a controller configured to:
   - detect a contact between an object and the first end, when the rebound tonometer is in use, by:
      - energizing the drive coil to move the magnetic elongated probe in respect to the body to have the first end at a second distance from the proximal end, the second distance being greater than the first distance;
      - measuring a first induced voltage in the measurement coil as a function of time during a first time period; and
      - comparing the measured first induced voltage as a function of time with a predetermined criterion, and if based on the comparison, the predetermined criterion is met, use it as an indication of a detected contact between the first end and the object; and
   - initiate a measurement cycle of the rebound tonometer when the contact is detected.

In another aspect, an embodiment of the present disclosure provides a method for using a rebound tonometer for determining a property of an object, wherein the rebound tonometer comprises a body, a magnetic elongated probe, a measurement coil and a drive coil, the method comprising:
- arranging the magnetic elongated probe at least partially in the body in a manner that a first end of the magnetic elongated probe protrudes outside of the body from an opening at a proximal end of the body, the first end is at a first distance from the opening, and a second end of the magnetic elongated probe is inside the body, wherein the magnetic elongated probe is arranged to be movable along an axis of the rebound tonometer;
- detecting a contact between the object and the first end by:
   - moving the magnetic elongated probe in respect to the body to have the first end at a second distance from the proximal end, the second distance being greater than the first distance;
   - moving the rebound tonometer in respect to the object during a first time period;
   - measuring a first induced voltage as a function of time during a first time period as the rebound tonometer is moved; and
   - comparing the measured first induced voltage as a function of time with a predetermined criterion, and if based on the comparison the predetermined criterion is met, using it as an indication of a detected contact between the first end and the object; and
- initiating a measurement cycle of the rebound tonometer when the contact is detected.

The present disclosure provides the aforementioned rebound tonometer and the aforementioned method. Herein, the controller of the rebound tonometer performs accurate alignment calibration of the rebound tonometer to obtain an optimal required distance of the object from the proximal end of the body in manner that when the measurement cycle of the rebound tonometer is initiated after the calibration, the property of the object is determined accurately and reliably. The measurement cycle is only initiated after the contact between the object and the first end is detected, to ensure that the object lies within the second distance from the proximal end of the body of the rebound tonometer. As a result, in the measurement cycle, the first end of the rebound tonometer just touches or collides with the object, without causing any accidental damage to the object. In other words, the magnetic elongated probe is ejected at a requisite distance which is neither too far from the object, nor too close to the object. This approach to ensure accurate alignment of the rebound tonometer with respect to the object prior to initiation of the measurement cycle is extremely simple, easy to implement, and inexpensive. Resultantly, examinations and/or diagnosis of the object using the rebound tonometer would be reliable, accurate, and error-free. Moreover, the rebound tonometer is light-weight and cost-effective. The method is fast, effective, reliable and can be performed easily.

Throughout the present disclosure, the term *"rebound tonometer"* refers to an instrument used for measuring the property of the object. Herein, the term *"property"* refers to a physiological parameter of the object. For example, the rebound tonometer may be used to measure physiological parameters of an eye such as, an intra-ocular pressure of the eye, a touch sensitivity of the eye, and the like. Optionally, the rebound tonometer is used for measuring intra-ocular pressure of the eye from an ophthalmic measurement.

The term *"body"* refers to an outermost physical structure of the rebound tonometer. The body is a housing wherein at least some components (such as the magnetic elongated probe, the measurement coil, the drive coil, and the like) of the rebound tonometer are arranged. In other words, the body is adapted to (partially or fully) accommodate the components of the rebound tonometer. It will be appreciated that the body has two opposite ends: the proximal end and the distal end. When the rebound tonometer is in use, the proximal end of the body is located in proximity of the object. The body of the rebound tonometer can be mounted on an apparatus or may be hand-held for utilisation of the tonometer. In some cases, the rebound tonometer is held manually by a user (for example, such as a medical professional). In such cases, the body is optionally provided with grooves to provide better grip the rebound tonometer manually, as compared to when such grooves are not provided. In other cases, the rebound tonometer is held using the apparatus such as a mechanical holder. The medical professional may, for example, be a doctor (such as an Ophthalmologist, Optometrist, General practitioner, and the like), a nurse, a medical support staff, or similar.

It will be appreciated that the components of the rebound tonometer could be arranged (namely, held or attached) in the body via adhesive means, mechanical means, magnetic means, and the like. In one case, the components of the rebound tonometer could be manufactured individually, and then these components could be assembled in the body. In another case, at least some of the components of the rebound tonometer could be manufactured as an integral part of the body.

The body has the opening at the proximal end. The opening allows the magnetic elongated probe to be arranged such that the magnetic elongated probe is moveable through the opening. Optionally, a shape and a size of the opening correspond to a shape and a size of the magnetic elongated probe. As an example, the magnetic elongated probe is cylindrical in shape and has a given radius, so the opening has a circular shape having a radius that is greater than the given radius.

Throughout the present disclosure, the term *"magnetic elongated probe"* refers to an elongated tool employed for determining the property of the object. The magnetic elongated probe is partially arranged within the opening of the body. It will be appreciated that the magnetic elongated probe has two opposite ends: the first end and the second end, such that the second end is arranged to be inside the body, and the first end protrudes outside of the body from the opening of the body. The magnetic elongated probe also has a middle section between the first end and the second end. Optionally, the first end of the magnetic elongated probe is made from bio-compatible material and will collide with a surface of the object (such as the eye) when in use. Beneficially, the first part being made of bio-compatible material enables the probe to function in intimate contact with living tissues of the eye, for example, causing minimal discomfort or pain. Notably, the bio-compatible material is free from carcinogenicity, toxicity, and is resistive to corrosion. As an example of arranging the magnetic elongated probe partially in the body is that the body an opening. The opening provides access to insert the magnetic elongated probe to a cylinder like cavity of the body. Diameter of the cylinder like cavity is larger than diameter of at least middle part and the second end of the elongated magnetic body, to enable movement of the elongated magnetic probe back and forth in the cavity. During use, the second end and initially about 80%-90% of the middle section is in the cavity. Reminding part of the middle section and the first end are (protrudes) outside of the body (via the opening). During measurement cycle the probe is ejected from the cavity of the body via the opening in such a way that 40-80% of the middle section is in the cavity and when the probe is its furthest distance from the opening of the body.

Furthermore, optionally, the elongated magnetic probe (or at least the middle section of the elongated magnetic probe) is made of a magnetic material. For example, the elongated magnetic probe may be made of a thin wire of magnetic material. Herein, the elongated magnetic probe may be, for example, 20 millimetres in length and 0.5 millimetre in width. Additionally, the magnetic material in the elongated magnetic probe may be ferromagnetic.

The first end and the proximal end are separated by the first distance in a default usage state the rebound tonometer. The default usage state may be one wherein the magnetic elongated probe is at rest (i.e., the magnetic elongated probe is not moving). For example, the first distance may lie in a range of 3-5 millimetres (mm) from the opening (towards an object i.e. outward from the opening of the body of the rebound tonometer when in use). The *"axis"* of the rebound tonometer refers to an imaginary line which passes along a length of the rebound tonometer and defines a path along which the magnetic elongated probe is aligned and is movable. It will be appreciated that the axis lies along a given geometrical direction. As an example, the optical axis may be a horizontal axis between an eye of the user of the rebound tonometer and the object.

The measurement coil is arranged inside the body and partially surrounds the magnetic elongated probe. Optionally, the measurement coil has a finite number of loops. The magnetic elongated probe may be arranged partially within a hollow space of the loops of the measurement coil. In such a case, the magnetic elongated probe may move through the loops of the measurement coil. Moreover, the movement of the magnetic elongated probe inside a measurement coil produces an induced voltage in the measurement coil.

The drive coil is also arranged inside the body and partially surrounds the magnetic elongated probe. Herein, the drive coil is arranged as a set of loops through which the elongated magnetic probe can move. Furthermore, the drive coil has a finite number of loops. The drive coil moves the elongated magnetic probe when electric current is fed through the drive coil. The drive coil optionally pulls the magnetic elongated probe and projects the magnetic elongated probe towards the surface of the object with a velocity which is equal to a product of the electric current fed through the drive coil times a magnetization of the magnetic elongated probe. Optionally, the drive coil is arranged as a set of loops through which the magnetic elongated probe can move. Furthermore, the drive coil has finite number of loops. Additionally, the drive coil may be arranged along any point between the first end and the second end. For instance, the drive coil may be arranged closer to the first end.

Optionally, the measurement coil and the drive coil are implemented as separate coils. In such a case, the measurement coil and the drive coil are physically separate from each other. Alternatively, optionally, the measurement coil and the drive coil are implemented as different portions of a single coil, such that one portion of the single coil is utilised as the measurement coil, a remaining portion of the single coil is utilised as the drive coil during operation of the rebound tonometer.

The term *"controller"* refers to a computational device that is operable for controlling the overall operation of the rebound tonometer. The controller, in operation, performs tasks such as, but not limited to, controlling movement of the magnetic elongated probe, and responding to and processing information. In an example, the controller may be an embedded microcontroller, a microprocessor, and the like. Optionally, the controller is coupled with the measurement coil and the drive coil. The controller may be implemented as an internal component of the rebound tonometer, an external component of the rebound tonometer, or a combination of these.

The contact between the object and the first end occurs upon a physical contact of the first end of the magnetic elongated probe with the object. The term *"object"* refers to an element of which a property is to be measured. Optionally, the object is a physical part of a body of an entity. The entity may, for example, be a human, an animal, or similar. For example, the object is the eye of a human. Beneficially, the contact between the object and the first end is detected prior to initialization of the measurement cycle, so that during the measurement cycle, the object lies within a requisite operational distance from the first end of the magnetic elongated probe. As a result, in the measurement cycle, the magnetic elongated probe is ejected to an appropriate distance so as to rebound from the surface of the object without damaging it and without causing discomfort, and thus enabling accurate measurement of the property of the object.

Optionally, the drive coil is energized by the controller by way of providing a current to the drive coil for magnetically energising the drive coil. When the drive coil is magnetically energised, it moves the magnetic elongated probe in respect of the body towards the object, such that the second distance between the first end of the magnetic elongated probe and the proximal end or opening of the body is greater than the first distance. Optionally, the second distance lies in a range of 6 mm - 8 mm. The second distance may, for example, be from 6, 6.2, 6.4, 6.6, 6.8, 7 or 7.5 mm up to 6.5, 7, 7.2, 7.4, 7.6, 7.8 or 8 mm. Optionally, the drive coil is magnetically energized such that at least a portion of the drive coil surrounding the second end of the magnetic elongated probe is positively charged with respect to the second end. Such magnetic energization of the drive coil repels the second end and projects the second end of the magnetic elongated probe towards a direction of the first end.

It will be appreciated that when the drive coil is energized, there is created a magnetic force to initiate movement of the magnetic elongated probe towards a direction of the first end. Herein, energizing refers to switching a supply voltage of the drive coil ON or OFF. Notably, an electric field is created when the supply voltage of the drive coil is switched ON. Typically, higher supply voltage of the drive coil will result in greater magnetic force. Subsequently, an acceleration of the magnetic elongated probe will increase. Moreover, the magnetic force is a function of magnetization (namely, magnetic polarization) of the magnetic elongated probe. Herein, the term magnetization refers to a density of dipole moment induced in the magnetic elongated probe. In particular, higher magnetization will result in greater magnetic force. Energizing the drive coil will move the magnetic elongated probe towards the direction of the first end (and particularly, towards the surface of the object). Moreover, the magnetic elongated probe will move in a reverse direction (i.e., away from the proximal end) in case polarity of the drive coil is reversed by energizing. The direction of moving the magnetic elongated probe is controlled by the controller.

The term *"first induced voltage"* refers to a voltage induced in the measurement coil due to a rebound movement of the magnetic elongated probe on contacting the object. Upon contacting the object, the first end of the magnetic elongated probe decelerates and then rebounds to move in the direction of the second end (i.e., towards the distal end of the body). As a result, the first induced voltage is induced in the measurement coil. The first induced voltage is an indication that a distance between the first end of the magnetic elongated probe and the object is less than or equal to the second distance. Herein, the measurement of the first induced voltage changes with a change in time as a velocity and an acceleration of the magnetic elongated probe changes during the first time period. It will be appreciated that the first time period refers to a time period of an alignment calibration cycle implemented prior to the measurement cycle, said time period extending from a start of a movement of the magnetic elongated probe in the direction of the first end for having the first end at the second distance from the proximal end, to an end of the movement of the magnetic elongated probe when the first end is again at the first distance from the proximal end.

Once the first end of the magnetic elongated probe is at the second distance from the proximal end of the body, the rebound tonometer and the object may be moved with respect to each other. If the relative arrangement of the object and the rebound tonometer is such that the object just contacts the first end, the object is determined to be at a distance equal to the second distance from the proximal end. However, it is possible that the relative arrangement of the object and the rebound tonometer is such that the magnetic elongated probe presses into the object when the first end is at the second distance from the proximal end. This indicates that the object is at a distance that is less than the second distance from the proximal end.

Throughout the present disclosure, the term *"predetermined criterion"* refers to a predefined standard which is utilized for making a decision. Optionally, the predetermined criterion is selected to be one of: a predetermined voltage threshold value, an integral value of the measured first induced voltage as function of time over the first time period, a derivate value of the first induced voltage as function of time or a predetermined pattern. The term *"predetermined voltage threshold value"* refers to a pre-known limit within which the measured first induced voltage is expected to remain when the first end of the magnetic elongated probe does not contact the object. When the probe contacts the object the voltage value is expected to be larger than the predetermined voltage threshold value. This value is a function of speed of collision with the object. The phrase *"integral value of the measured first induced voltage as function of time over the first time period"* refers to an expected cumulative value of the measured first induced voltage over the first time period when the first end of the magnetic elongated probe does not contact the object during the first time period. Since the induced voltage is a function of speed the integral value is integral value of the speed in practical terms. The integral value is beneficial to use since the speed of the probe, when using the integral value, does not need to exceed certain threshold. The integral value gives indication if the probe has moved from its initial position independently on the speed thus providing means for detecting slow movement of probe towards the object. Further benefit of using integral value is that threshold value can be set higher. The phrase *"derivate value of the first induced voltage as function of time"* refers to an expected change of the measured first induced voltage over time. Usage of derivate can increase sensitivity of the triggering as it is related to change of speed (acceleration). Derivate value can be also used to filter away very fast accelerations caused for example of random vibrations of the device (such as caused by shaking hand of the doctor). The term *"predetermined pattern"* refers to a pre-known pattern of values, and since the comparison of the predetermined criterion is done with the measured induced voltage, the predetermined pattern optionally refers to a pattern of voltage values over time.

By the phrase *"predetermined criterion is met",* it is meant that one of the following occurs:
- the measured first induced voltage exceeds the predetermined voltage threshold value;
- a calculated integral value obtained upon evaluating an integral of the measured first induced voltage as function of time over the first time period is greater than the integral value;
- a calculated derivate value obtained upon evaluating a derivative of the measured induced voltage as the function of time exceeds the derivate value; or
- a pattern of values of the measured first induced voltage over time is similar to the predetermined pattern (of voltage values over time).

It will be appreciated that meeting of the predetermined criteria indicates that the first end contacts the object, which means that the object lies within or at a required operational distance from the proximal end of the body. In such a case, the required operational distance is the second distance. The measurement cycle of the rebound tonometer is initiated when the contact between the object and the first end is detected. If such a contact is not detected, the measurement cycle is not initiated.

The term *"measurement cycle"* refers to an operational cycle of ejecting the magnetic elongated probe with magnetic force to make it collide with the object with the purpose of measuring the property of the object. During the measurement cycle, the rebound magnetic elongated probe is retracted into the body so that the rebound tonometer is in its default usage state, and is then ejected out of the body to have the first end at the third distance from the proximal end. The magnetic elongated probe bounces back from the object after hitting the object. During the movement (ejecting, touching the object and bouncing back from the object) the magnetic elongated body induces, in an measurement coil, a second voltage as function of time. The second voltage as function of time is indication of speed of the probe as function of time (since varying magnetic field induces voltage in a coil). The speed as function of time can be used to determine property of object such as its elasticity, or in case of eye intra ocular pressure. It will be appreciated that the measurement cycle is initiated for determining the property of the object. The measurement cycle might be a single measurement or the measurement cycle might be repeated several times.

Optionally, when initiating the measurement cycle of the rebound tonometer, the controller is configured to:
- energize the measurement coil to retract the magnetic elongated probe into the body to have the first end at the first distance from the proximal end; and
- energize the drive coil to eject the magnetic elongated probe out of the body to have the first end at a third distance from the proximal end.

In this regard, by energizing the measurement coil and the drive coil one after the other, there is created a magnetic force for facilitating movement of the magnetic elongated probe firstly into the body and then out of the body. Firstly, at least one portion of the measurement coil surrounding the second end is optionally charged negatively with respect to the magnetic elongated probe for retracting the magnetic elongated probe into the body to have the first end at the first distance from the proximal end, such that the rebound tonometer is at the default usage state. Next, at least one portion of the drive coil surrounding the magnetic elongated probe is optionally charged positively with respect to the second end for ejecting the magnetic elongated probe out of the body to have the first end at the third distance from the proximal end. In such a case, the magnetic elongated probe is accelerated by a current pulse in the drive coil, which generates a magnetic field acting on the magnetic elongated probe.

Optionally, the third distance lies in a range of 0.5 to 2.0 times of the second distance. In an example, the third distance may lie in a range of 0.5, 0.6, 0.7, 0.8, 1.0, 1.2, 1.5 or 1.8 times of the second distance to 1.3, 1.5, 1.8, 1.9 or 2.0 times of the second distance. For example, if the second distance is 6 mm, the third distance may lie in a range of 4.8 mm -12 mm. As an example, the third distance may be 7.5 mm.

In an embodiment, when the distance of the object from the proximal end is equal to the second distance, the controller is further configured to:
- select the third distance to be equal to or greater than the second distance; and
- complete the measurement cycle of the rebound tonometer by measuring a second voltage that is induced in the measurement coil as a function of time during a second time period of the ejection of the magnetic elongated probe, wherein the second voltage is indicative of collision of the first end with the object.

Herein, when the third distance is selected to be equal to the second distance, the first end just contacts the object; whereas when the third distance is selected to be greater than the second distance, the first end contacts the object with full force. In both these scenarios, the first end would rebound upon contacting the object, which in turn induces the second voltage in the measurement coil. The measurement cycle, therefore, is completed by measuring the second voltage that is induced in the measurement coil. The second time period refers to a time period of the measurement cycle, said time period extending from a start of a movement of the magnetic elongated probe in the direction of the first end for having the first end at the third distance from the proximal end, to an end of the movement of the magnetic elongated probe when the first end is again at the first distance from the proximal end.

Optionally, when the selected third distance is greater than 2 times of the second distance, the controller is further configured to provide an indication to increase the distance between the object and the proximal end of the body on a user interface of the rebound tonometer. The selected third distance being greater than 2 times of the second distance means that the third distance may be at least twice of the second distance. For example, if the second distance is 6 mm, the selected third distance may be more than 12 mm. In such a case, the magnetic elongated probe could to collide too hard with the object, which is undesirable as such collision may harm the object and/or the magnetic elongated probe. Herein, the indication is provided to enable for example, the user of the rebound tonometer, to increase the distance between the object and the proximal end of the body. Such indication to increase the distance is provided for preventing the rebound magnetic elongated probe from colliding too hard with the object (which may be a sensitive object such as an eye). When the indication is observed, the distance is increased by movement of the rebound tonometer or movement of the object. Such movement may be manual and may be performed by the user of the rebound tonometer. Upon increasing the distance, a force with which the magnetic elongated probe collides with the object is reduced, thereby preventing any damage to the object and/or the magnetic elongated probe.

The term *"user interface"* refers to an interface via which the user interacts with the rebound tonometer. The user interface is implemented on at least one of: the rebound tonometer, a computing device coupled to the rebound tonometer. Examples of the computing device include, but are not limited to, a computer, a smartphone, a smartwatch, a tablet, and a laptop. Optionally, the user interface is rendered on a display of the rebound tonometer or the computing device. In this regard, the indication could be a text indication, an image indication, an audio-visual indication, an error notification, a warning or similar. Alternatively, the indication may be provided using at least one of: an audio device, a light-emitting diode (LED), a vibration device, a haptic device, associated with the rebound tonometer. As an example, the controller may provide the indication by way of controlling a vibration device installed in the rebound tonometer to vibrate profusely when the selected third distance is greater than 2 times of the second distance. As another example, the controller may provide the indication by way of controlling an LED installed in the rebound tonometer to emit red light when the selected third distance is greater than 2 times of the second distance.

In another embodiment, when the distance of the object from the proximal end is less than the second distance, the controller is further configured to:
- select the third distance to be less than the second distance; and
- complete the measurement cycle of the rebound tonometer by measuring a second voltage that is induced in the measurement coil as a function of time during a second time period of the ejection of the magnetic elongated probe, wherein the second voltage is indicative of collision of the first end with the object.

Herein, the third distance is selected to be less than the second distance for preventing the rebound magnetic elongated probe from colliding too hard with the object. In such a scenario, the third distance may be so selected to account for a possibility that the user may still move the rebound tonometer closer to the object or the object may still move closer to the rebound tonometer, after initiation of the measurement cycle. A technical benefit of selecting the third distance to be less than the second distance in this embodiment ensures that the first end contacts the object with requisite force for completing the measurement cycle without harming the object during the same. The first end would rebound upon contacting the object, which in turn induces the second voltage in the measurement coil. The measurement cycle, therefore, is completed by measuring the second voltage that is induced in the measurement coil.

Optionally, the controller is further configured to use the second voltage to determine a property of the object. The term *"property"* refers to a physiological parameter of the object. For example, the rebound tonometer may be used to measure a physiological parameter of an eye such as, intra-ocular pressure of the eye, touch sensitivity (of eye or skin) and the like. For example, the rebound tonometer may be used for measuring an intra-ocular pressure of the eye from an ophthalmic measurement. The second voltage represents velocity as a function of time of the magnetic elongated probe that rebounds from the object when the first end of the magnetic elongated probe collides with (the surface of) the object. The magnitude of the second voltage depends on the magnetic strength and speed of the magnetic elongated probe. For example, the magnitude of the second voltage is directly proportional to the speed of the magnetic elongated probe. In an example, the second voltage may be used to determine an intra-ocular pressure of an eye. The speed of the magnetic elongated probe and a response of a surface of the eye to stop the magnetic elongated probe once it is ejected towards and collides with the surface of the eye, may be determined by calculating first derivate of the velocity. Furthermore, the speed of the magnetic elongated probe rebounding from the surface of the eye may be determined. Herein, the intra-ocular pressure is determined to be high in case the magnetic elongated probe rebounds rapidly (i.e., at a high speed), and the intra-ocular pressure is low in case the magnetic elongated probe rebounds slowly (i.e., at a low speed).

Optionally, the rebound tonometer further comprises at least one sensor, wherein the controller is configured to:
- collect, from the at least one sensor, sensor data indicative of displacement and/or velocity of the magnetic elongated probe as a function of time during the measurement cycle;
- determine a velocity and/or an acceleration of the magnetic elongated probe, based on the sensor data; and
- determine a property of the object, based on a change in the velocity and/or the acceleration of the magnetic elongated probe.

Optionally, in this regard, the sensor measures the displacement and/or velocity of the magnetic elongated probe and records the sensor data related thereto. A movement of the magnetic elongated probe may be expressed in terms of the displacement and/or velocity of the magnetic elongated probe as the function of time. In an instance, the sensor is configured to measure a change in magnetic flux in the measurement coil due to a movement of the magnetic elongated probe towards the object. As mentioned previously, during the measurement cycle, magnetic force is exerted to eject the magnetic elongated probe out of the body to collide with the object, after which the magnetic elongated probe retracts into the body. Therefore, the sensor measures the changes in the magnetic flux in the measurement coil and the controller uses such measurement to determine the displacement and/or velocity of the probe. Optionally, the sensor is implemented using a transducer, an accelerometer, a frequency sensor, laser surface velocimeter, a piezoelectric sensor.

Optionally, when determining the property of the object based on the change in the velocity and/or the acceleration of the magnetic elongated probe, the controller compares the velocity and/or the acceleration of the magnetic elongated probe with historically-collected velocity and/or acceleration data of medical trials. For example, if the property is the intra-ocular pressure of the eye, a high value of the velocity and/or the acceleration would mean a high intra-ocular pressure, and *vice versa,* since the intra-ocular pressure is historically known to be high in case the magnetic elongated probe rebounds rapidly (i.e., with high velocity and/or acceleration), and the intra-ocular pressure is historically known to be low in case the magnetic elongated probe rebounds slowly. Beneficially, determination of the property of the object allows further diagnostic progress pertaining to the object.

Optionally, the controller is further configured to compare the measured first induced voltage as function of time with a second predetermined criterion, and if based on the comparison the second predetermined criterion is met, use it as an indication to provide a warning indicator on a user interface of the rebound tonometer. Optionally, the second predetermined criterion is selected to be one of: a second predetermined voltage threshold value, a second integral value of the measured first induced voltage as function of time over the first time period, a second derivate value of the first induced voltage as function of time or a second predetermined pattern. The term *"second predetermined voltage threshold value"* refers to a second pre-known limit within which the measured first induced voltage is expected to remain when the first end of the magnetic elongated probe does not contact the object. The phrase *"second integral value of the measured first induced voltage as function of time over the first time period"* refers to a second expected cumulative value of the measured first induced voltage over the first time period when the first end of the magnetic elongated probe does not contact the object during the first time period. The phrase *"second derivate value of the first induced voltage as function of time"* refers to an expected second change of the measured first induced voltage over time. The term *"second predetermined pattern"* refers to a pre-known second pattern of values, and since the comparison of the predetermined criterion is done with the measured induced voltage, the second predetermined pattern optionally refers to a second pattern of voltage values over time.

By the phrase *"second predetermined criterion is met",* it is meant that one of the following occurs:
- the measured first induced voltage exceeds the second predetermined voltage threshold value;
- a calculated integral value obtained upon evaluating an integral of the measured first induced voltage as function of time over the first time period is greater than the second integral value;
- a calculated derivate value obtained upon evaluating a derivative of the measured induced voltage as the function of time exceeds the second derivate value; or
- a pattern of values of the measured first induced voltage over time is similar to the second predetermined pattern (of voltage values over time).

It will be appreciated that when the measured first induced voltage exceeds the second predetermined voltage threshold value, it indicates that the rebound tonometer is being moved too quickly towards the object, which may harm the object. The calculated derivative value exceeding the second derivate value, too, implies (i.e., indicates) that the rebound tonometer is being moved too quickly towards the object. The calculated integral being greater than the second integral value implies that the first end is too close to the object. Similarly, the pattern of values of the measured first induced voltage being similar to the second predetermined pattern implies that the first end is too close to the object. Therefore, meeting of the second predetermined criterion indicates that the rebound tonometer is moving in an undesirable, unsafe manner.

The term *"warning indicator"* refers to an indication provided to the user of the rebound tonometer, when the measured first induced voltage as the function of time meets the second predetermined criterion, to indicate that the rebound tonometer is moving in the undesirable, unsafe manner. Upon observing the warning indicator, the user of the rebound tonometer may take corrective actions (such as de-energizing the drive coil, moving the object away from the rebound tonometer, and so forth) to prevent damage to the object. Notably, the warning indicator is provided to the user via the user interface. The warning indicator may be in form of a text indication, a visual indication, an audio-visual indication, or similar. Alternatively, the warning indicator may be provided on at least one of: the audio device, the light-emitting device (LED), the vibration device, the haptic device, associated with the rebound tonometer. As another example, the controller may provide the warning indicator by way of controlling an LED installed in the rebound tonometer to emit blue light when the measured first induced voltage as the function of time meets the second predetermined criterion.

Optionally, the controller is further configured to display an intensity of a given voltage with respect to a given time as a waveform on at least one display, a given display being at least one of: a display of an oscilloscope, a display of the rebound tonometer, a display of a computing device. The *"intensity"* of the given voltage refers to a measure of the given voltage. In other words, the intensity represents how high or how low the voltage is at the given time. It will be appreciated that the waveform representing the intensity of the given voltage with respect to time is displayed on the at least one display, to allow the user of the rebound tonometer to easily view and identify when the given voltage is induced, and to easily view the intensity of the given voltage. The user, then, can make alterations to a relative arrangement of the rebound tonometer and the object, provide diagnostics based on the intensity, or similar.

The present disclosure also relates to the method as described above. Various embodiments and variants disclosed above, with respect to the aforementioned first aspect, apply *mutatis mutandis* to the method.

Optionally, in the method, the predetermined criterion is selected to be one of: a predetermined voltage threshold value, an integral value of the measured first induced voltage as function of time over the first time period, a derivate value of the first induced voltage as function of time or a predetermined pattern.

Optionally, in the method, the measurement cycle comprises:
- energizing a measurement coil to retract the magnetic elongated probe into the body to have the first end at the first distance from the proximal end; and
- energizing the drive coil to eject the magnetic elongated probe out of the body to have the first end at a third distance from the proximal end;
- completing the measurement cycle by measuring a second voltage that is induced in the measurement coil as a function of time during a second time period of the ejection of the magnetic elongated probe, wherein the second voltage is indicative of the first end colliding with the object; and
- using the second voltage for determining the property of the object.

The second voltage is an indication of speed of the elongated probe as function of time. This indication can be used to determine property of an object. As an example if the second voltage is high it indicates that probe rebounds from the object in rapid manner thus the object is more stiff than in case it would rebound slower (low second voltage). The rebound tonometer can be used for example determining intraocular pressure of an eye (as one example of an measurement cycle). Other example of measurement cycle is to use rebound tonometer to collide with skin of a target user to determine touch sensitivity of the skin.

Optionally, in the method, the third distance lies in a range of 0.5 to 2.0 times of the second distance.

Optionally, when the distance of the object from the proximal end is equal to the second distance, the method further comprises selecting the third distance to be equal to or greater than the second distance.

Optionally, when the third distance is greater than 2 times of the second distance, the method further comprises providing an indication to increase the distance between the object and the proximal end of the body on a user interface of the rebound tonometer.

Optionally, when the distance of the object from the proximal end is less than the second distance, the method further comprises selecting the third distance to be less than the second distance.

Optionally, the method further comprises:
- collecting, from at least one sensor of the rebound tonometer, sensor data indicative of displacement and/or velocity of the magnetic elongated probe as a function of time during the measurement cycle;
- determining a velocity and/or an acceleration of the magnetic elongated probe, based on the sensor data; and
- determining a property of the object, based on a change in the velocity and/or the acceleration of the magnetic elongated probe.

Optionally, the method further comprises comparing the measured first induced voltage as function of time with a second predetermined criterion, and if based on the comparison the second predetermined criterion is met, using it as an indication to provide a warning indicator on a user interface of the rebound tonometer.

Optionally, in the method, the second predetermined criterion is selected to be one of: a second predetermined voltage threshold value, a second integral value of the measured first induced voltage as function of time over the first time period, a second derivate value of the first induced voltage as function of time or a second predetermined pattern.

As a further example the present disclosure provides a rebound tonometer which detects automatically distance between the rebound tonometer and an object and can thus initiate measurement cycle automatically. This (detection of distance) is important as if the distance is too low the probe might hit the object (such as eye) too fast and cause damage. Furthermore if the measurement cycle is initiated out too far away from the object the probe might hit the object too slow or might not hit the object at all. Distance of the object is detected by moving the first end of the probe to a distance from the body (towards the object) and allowing the first end of the probe to be in contact with the object. Since the probe is arranged to move in the body (opening / cavity / channel of the body) and it is surrounded with measurement coil the contact can be detected. Induced voltage to the measurement coil is used to initiate the measurement cycle.

### DETAILED DESCRIPTION OF THE DRAWINGS

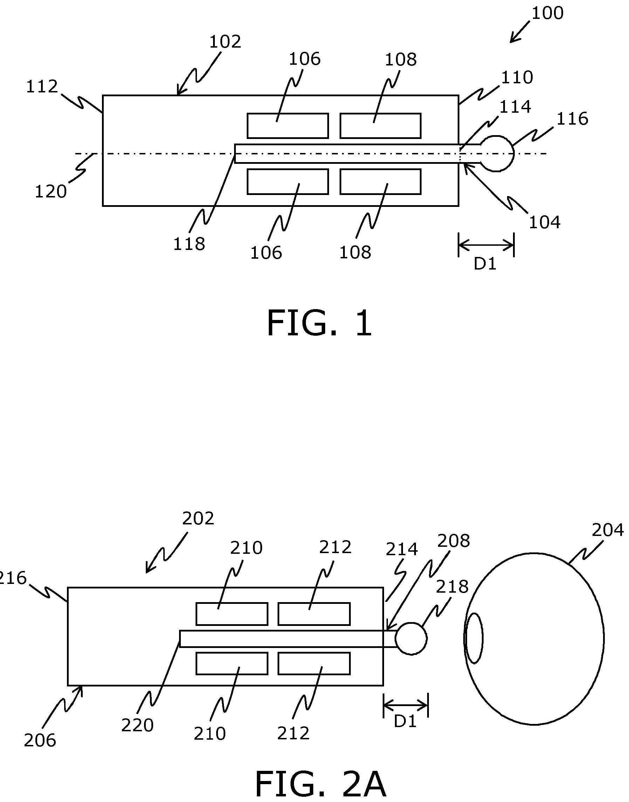

Referring to FIG. 1, illustrated is an exemplary schematic diagram of a rebound tonometer **100,** in accordance with an embodiment of the present disclosure. The rebound tonometer **100** comprises a body **102,** a magnetic elongated probe **104,** a measurement coil **106,** a drive coil **108** and a controller (not shown). The body **102** has a proximal end **110** and a distal end **112** opposite to the proximal end **110.** Moreover, the body **102** has an opening **114** (shown as a dotted line) at the proximal end **110.** The magnetic elongated probe **104** has a first end **116** and a second end **118** opposite to the first end **116,** such that the magnetic elongated probe **104** is arranged at least partially in the body **102** in a manner that the first end **116** protrudes outside of the body **102** from the opening **114** of the body **102.** The measurement coil **106** and the drive coil **108** are arranged inside the body **102** partially surrounding the magnetic elongated probe **104.** The controller is coupled to the measurement coil **106** and the drive coil **108.** The first end **116** is at a first distance **D1** from the opening **114,** and the second end **118** is inside the body **102,** wherein the magnetic elongated probe **104** is arranged to be aligned with and movable along an axis **120** (shown as a dashed dot line) of the rebound tonometer **100.**

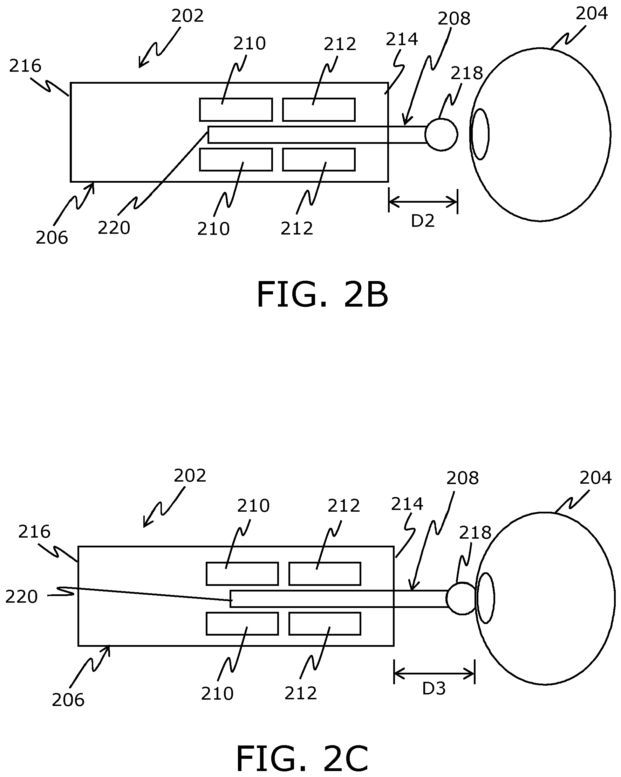

Referring to FIGs. 2A, 2B and 2C, illustrated are exemplary schematic diagrams of usage states of a rebound tonometer **202,** in accordance with various embodiments of the present disclosure. The rebound tonometer **202** is used to determine a property of an object **204** (depicted as an eye). The rebound tonometer **202** comprises a body **206,** a magnetic elongated probe **208,** a measurement coil **210,** a drive coil **212** and a controller (not shown). The body **206** has a proximal end **214** and a distal end **216** opposite to the proximal end **214.** Moreover, the body **206** has an opening (not shown) at the proximal end **214.** The magnetic elongated probe **208** has a first end **218** and a second end **220** opposite to the first end **218,** such that the magnetic elongated probe **208** is arranged at least partially in the body **206** in a manner that the first end **218** protrudes outside of the body **206** from the opening of the body **206.** The measurement coil **210** and the drive coil **212** are arranged inside the body **206** partially surrounding the magnetic elongated probe **208.** The controller is coupled to the measurement coil **210,** and the drive coil **212.**

In FIG. 2A, the rebound tonometer **202** is shown to be in a first usage state (for example, such as a rest condition). At the rest condition, the first end **218** is at a first distance **D1** from the opening, and the second end **220** is inside the body **206.** The magnetic elongated probe **208** is arranged to be aligned with and movable along an axis (not shown) of the rebound tonometer **202.** Herein, the first end **218** is away from (i.e., does not contact) the object **204.**

In FIG. 2B, the rebound tonometer **202** is shown to be in a second usage state. To transition from the first usage state to the second usage state, the controller energizes the drive coil **212** to move the magnetic elongated probe **208** with respect to the body **206** to have the first end **218** at a second distance **D2** from the proximal end **214.** The second distance **D2** is greater than the first distance **D1** (shown in FIG. 2A). In the second usage state, the first end **218** of the rebound tonometer **202** is closer to the object **204** as compared to the first usage state shown in FIG. 2A. In FIG. 2B, there is depicted an instance wherein a distance of the object **204** from the proximal end **214** is greater than the second distance **D2.** However, when the distance of the object **204** from the proximal end **214** is equal to or less than the second distance **D2**, in the second usage state, the first end **218** contacts the object **204.** Upon detection of the contact between the object **204** and the first end **218,** a measurement cycle of the rebound tonometer **202** is initiated.

In FIG. 2C, the rebound tonometer **202** is shown to be at a third usage state. To transition from the first usage state to the third usage state, the controller energizes the drive coil **212** to eject the magnetic elongated probe **208** out of the body **206** to have the first end **218** at a third distance **D3** from the proximal end **214.** Here, the third distance **D3** is shown to be greater than the second distance **D2**. In this case, the first end **218** contacts the object **204** with full force. The measurement cycle of the rebound tonometer **202** is completed by measuring a second voltage that is induced in the measurement coil **210** as a function of time during a second time period of the ejection of the magnetic elongated probe **208,** wherein the second voltage is indicative of collision of the first end **218** with the object **204.**

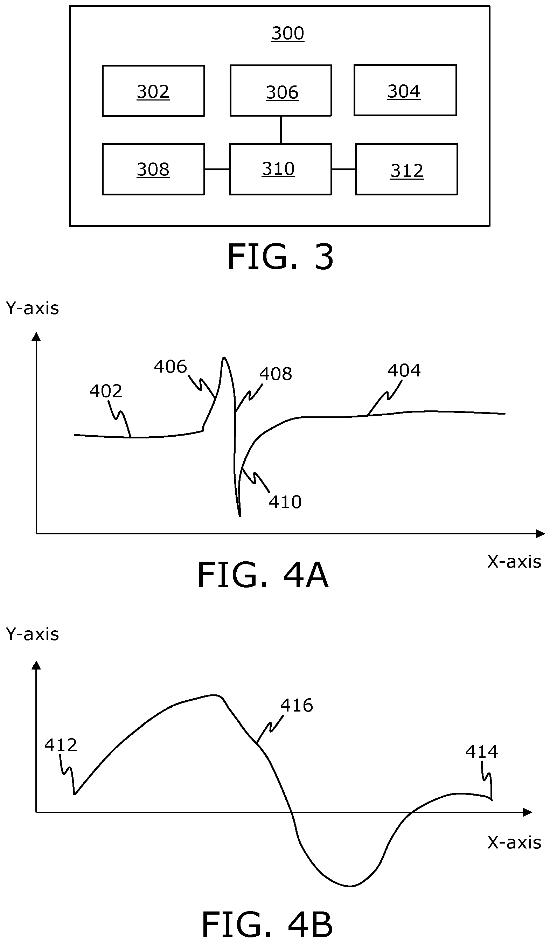

Referring to FIG. 3, illustrated is a block diagram of a rebound tonometer **300,** in accordance with an embodiment of the present disclosure. The rebound tonometer **300** comprises a body **302,** a magnetic elongated probe **304,** a measurement coil **306,** a drive coil **308,** a controller **310,** and at least one sensor (depicted as a sensor **312).** The controller **310** is coupled to the measurement coil **306,** the drive coil **308** and the sensor **312.**

FIGs. 1, 2A-2C, and 3 are merely examples, which should not unduly limit the scope of the claims herein. It will be appreciated that the rebound tonometers **100, 202** and **300** are provided as examples and are not to be construed as limiting the same to specific numbers or types of components. A person skilled in the art will recognize many variations, alternatives, and modifications of embodiments of the present disclosure.

Referring to FIGs. 4A and 4B, FIG. 4A illustrates a first waveform of a first induced voltage in a measurement coil as a function of time during a first time period, while FIG. 4B illustrates a second waveform of a second voltage that is induced in the measurement coil as a function of time during a second time period, in accordance with an embodiment of the present disclosure. A given waveform represents an value/amplitude of a given voltage with respect to time. Herein, Y-axis represents the intensity of the given voltage, while X-axis represents time.

In FIG. 4A, portions **402** and **404** of the first waveform indicate a (nearly) constant intensity of voltage through their corresponding time. This means that no voltage is induced in the measurement coil during the time corresponding to the portions **402** and **404.** Portions **406, 408,** and **410** (depicted as a crest and a trough) in the first waveform represents the first induced voltage. For example, the portion **406** may correspond to a time of contact of a first end of a magnetic elongated probe with an object wherein the first end pushes into the object, the portion **408** may correspond to a time wherein the first end is moving away from the object but is still in contact with the object, and the portion **410** may correspond to a time wherein the first end is moving away from the object and is no longer in contact with the object.

In FIG. 4B, a point **412** in the second waveform indicates a time at which the first end is at a first distance from an opening in a proximal end of a body of a rebound tonometer. At the point **412,** no voltage is induced in the measurement coil. A point **414** in the second waveform indicates another time at which the first end is at the first distance from the opening. At the point **414,** no voltage is induced in the measurement coil.

A portion **416** of the second waveform that lies between the point **412** and the point **414** indicates the second voltage that is induced in the measurement coil. The second voltage varies as a function of time, as depicted by a crest and a trough in the portion **416,** and is induced when a magnetic elongated probe is ejected out of the body to have the first end at a third distance from the proximal end, collides with the object, and rebounds upon collision with the object.

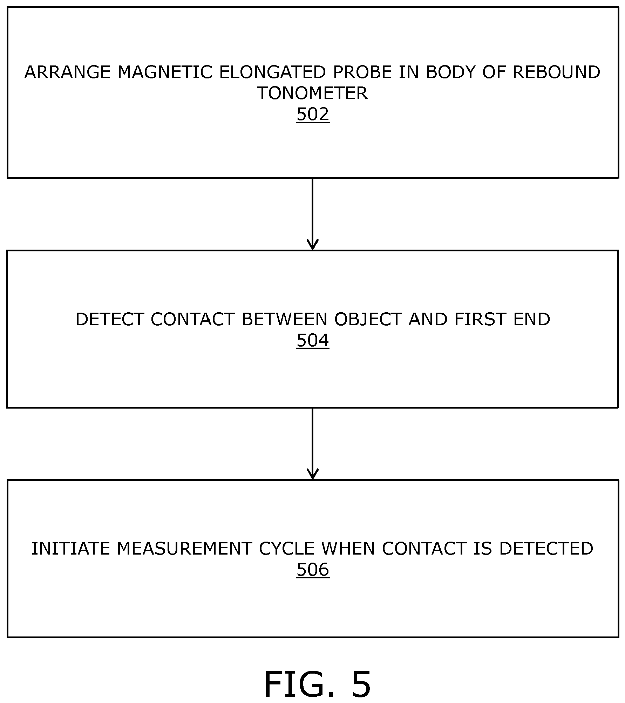

Referring to FIG. 5, illustrated is a flowchart depicting steps of a method for using a rebound tonometer for determining a property of an object, in accordance with an embodiment of the present disclosure. The rebound tonometer comprises a body, a magnetic elongated probe, a measurement coil and a drive coil. At step **502,** the magnetic elongated probe is arranged at least partially in the body in a manner that a first end of the magnetic elongated probe protrudes outside of the body from an opening at a proximal end of the body, the first end being at a first distance from the opening, and a second end of the magnetic elongated probe being inside the body, wherein the magnetic elongated probe is arranged to be movable along an axis of the rebound tonometer. At step **504,** a contact between an object and the first end is detected. The detection is done by moving the magnetic elongated probe in respect to the body to have the first end at a second distance from the proximal end, the second distance being greater than the first distance; moving the rebound tonometer in respect to the object during a first time period, measuring a first induced voltage as a function of time during a first time period as the rebound tonometer is moved, and comparing the measured first induced voltage as a function of time with a predetermined criterion, and if based on the comparison the predetermined criterion is met, using it as an indication of a detected contact between the first end and the object. At step **506,** a measurement cycle of the rebound tonometer is initiated when the contact is detected.

The steps **502, 504,** and **506** are only illustrative and other alternatives can also be provided where one or more steps are added, one or more steps are removed, or one or more steps are provided in a different sequence without departing from the scope of the claims herein.

## Claims

1. A rebound tonometer (100, 202, 300) comprising:
a body (102, 206, 302) having a proximal end (110, 214) and a distal end (112, 216) opposite to the proximal end, wherein the body has an opening (114) at the proximal end;
a magnetic elongated probe (104, 208, 304) having a first end (116, 218) and a second end (118, 220) opposite to the first end, wherein the magnetic elongated probe is arranged at least partially in the body in a manner that the first end protrudes outside of the body from the opening of the body, the first end is at a first distance (D1) from the opening, and the second end is inside the body, and wherein the magnetic elongated probe is arranged to be aligned with and movable along an axis (120) of the rebound tonometer;
a measurement coil (106, 210, 306) and a drive coil (108, 212, 308) arranged inside the body and to partially surround the magnetic elongated probe; and
a controller (310) configured to:
- detect a contact between an object (204) and the first end, when the rebound tonometer is in use, by:
- energizing the drive coil to move the magnetic elongated probe in respect to the body to have the first end at a second distance (D2) from the proximal end, the second distance being greater than the first distance;
- measuring a first induced voltage in the measurement coil as a function of time during a first time period, and
- comparing the measured first induced voltage as function of time with a predetermined criterion, and if based on the comparison the predetermined criterion is met, use it as an indication of a detected contact between the first end and the object; and
- initiate a measurement cycle of the rebound tonometer when the contact is detected.

2. A rebound tonometer (100, 202, 300) of claim 1, wherein the predetermined criterion is selected to be one of: a predetermined voltage threshold value, an integral value of the measured first induced voltage as function of time over the first time period, a derivate value of the first induced voltage as function of time or a predetermined pattern.

3. A rebound tonometer (100, 202, 300) of claim 1 or 2, wherein when initiating the measurement cycle of the rebound tonometer, the controller (310) is configured to:
- energize the measurement coil (106, 210, 306) to retract the magnetic elongated probe (104, 208, 304) into the body (102, 206, 302) to have the first end (116, 218) at the first distance (D1) from the proximal end (110, 214); and
- energize the drive coil (108, 212, 308) to eject the magnetic elongated probe out of the body to have the first end at a third distance (D3) from the proximal end.

4. A rebound tonometer (100, 202, 300) of claim 3, wherein the third distance lies in a range of 0.5 to 2.0 times of the second distance.

5. A rebound tonometer (100, 202, 300) of claim 3 or 4, wherein when the distance of the object (204) from the proximal end (110, 214) is equal to the second distance, the controller (310) is further configured to:
- select the third distance to be equal to or greater than the second distance; and
- complete the measurement cycle of the rebound tonometer by measuring a second voltage that is induced in the measurement coil (106, 210, 306) has a function of time during a second time period of the ejection of the magnetic elongated probe (104, 208, 304), wherein the second voltage is indicative of collision of the first end (116, 218) with the object.

6. A rebound tonometer (100, 202, 300) of claim 5, wherein when the selected third distance is greater than 2 times of the second distance, the controller (310) is further configured to provide an indication to increase the distance between the object (204) and the proximal end (110, 214) of the body (102, 206, 302) on a user interface of the rebound tonometer.

7. A rebound tonometer (100, 202, 300) of claim 3 or 4, wherein when the distance of the object (204) from the proximal end (110, 214) is less than the second distance, the controller (310) is further configured to:
- select the third distance to be less than the second distance; and
- complete the measurement cycle of the rebound tonometer by measuring a second voltage that is induced in the measurement coil (106, 210, 306) as a function of time during a second time period of the ejection of the magnetic elongated probe (104, 208, 304), wherein the second voltage is indicative of collision of the first end (116, 218) with the object.

8. A rebound tonometer (100, 202, 300) of any of claims 5-7, wherein the controller (310) is further configured to use the second voltage to determine a property of the object (204).

9. A rebound tonometer (100, 202, 300) of any of the preceding claims, further comprising at least one sensor (312), wherein the controller (310) is configured to:
- collect, from the at least one sensor, sensor data indicative of displacement and/or velocity of the magnetic elongated probe (104, 208, 304) as a function of time during the measurement cycle;
- determine a velocity and/or an acceleration of the magnetic elongated probe, based on the sensor data; and
- determine a property of the object (204), based on a change in the velocity and/or the acceleration of the magnetic elongated probe.

10. A rebound tonometer (100, 202, 300) of any of the preceding claims, wherein the controller (310) is further configured to compare the measured first induced voltage as function of time with a second predetermined criterion, and if based on the comparison the second predetermined criterion is met, use it as indication to provide a warning indicator on a user interface of the rebound tonometer.

11. A rebound tonometer (100, 202, 300) of claim 10, wherein the second predetermined criterion is selected to be one of: a second predetermined voltage threshold value, a second integral value of the measured first induced voltage as function of time over the first time period, a second derivate value of the first induced voltage as function of time or a second predetermined pattern.

12. A method for using a rebound tonometer (100, 202, 300) for determining a property of an object (204), wherein the rebound tonometer comprises a body (102, 206, 302), a magnetic elongated probe (104, 208, 304), a measurement coil (106, 210, 306) and a drive coil (108, 212, 308), the method comprising:
- arranging the magnetic elongated probe at least partially in the body in a manner that a first end (116, 218) of the magnetic elongated probe protrudes outside of the body from an opening (114) at a proximal end (110, 214) of the body, the first end is at a first distance from the opening, and a second end (118, 220) of the magnetic elongated probe is inside the body, wherein the magnetic elongated probe is arranged to be movable along an axis (120) of the rebound tonometer;
- detecting a contact between of the object and the first end by:
- moving the magnetic elongated probe in respect to the body to have the first end at a second distance from the proximal end, the second distance being greater than the first distance;
- moving the rebound tonometer in respect to the object during a first time period;
- measuring a first induced voltage as a function of time during a first time period as the rebound tonometer is moved, and
- comparing the measured first induced voltage as function of time with a predetermined criterion, and if based on the comparison the predetermined criterion is met, using it as indication of a detected contact between the first end and the object;
- initiating a measurement cycle of the rebound tonometer when the contact is detected.

13. A method according to claim 12, wherein the predetermined criterion is selected to be one of: a predetermined voltage threshold value, an integral value of the measured first induced voltage as function of time over the first time period, a derivate value of the first induced voltage as function of time or a predetermined pattern.

14. A method according to claim 12 or 13, wherein the measurement cycle comprises:
- energizing a measurement coil (106, 210, 306) to retract the magnetic elongated probe (104, 208, 304) into the body (102, 206, 302) to have the first end (116, 218) at the first distance from the proximal end (110, 214);
- energizing the drive coil (108, 212, 308) to eject the magnetic elongated probe out of the body to have the first end at a third distance from the proximal end;
- completing the measurement cycle by measuring a second voltage that is induced in the measurement coil as a function of time during a second time period of the ejection of the magnetic elongated probe, wherein the second voltage is indicative of the first end colliding with the object (204); and
- using the second voltage for determining the property of the object.

15. A method according to claim 14, wherein when the distance of the object (204) from the proximal end (110, 214) is equal to the second distance, the method further comprises selecting the third distance to be equal to or greater than the second distance.

16. A method according to claim 15, wherein when the third distance is greater than 2 times of the second distance, the method further comprises providing an indication to increase the distance between the object (204) and the proximal end (110, 214) of the body (102, 206, 302) on a user interface of the rebound tonometer (100, 202, 300).

17. A method according to claim 14, wherein when the distance of the object (204) from the proximal end (110, 214) is less than the second distance, the method further comprises selecting the third distance to be less than the second distance.

18. A method according to any of claims 12-17, further comprising:
- collecting, from at least one sensor (312) of the rebound tonometer (100, 202, 300), sensor data indicative of displacement and/or velocity of the magnetic elongated probe (104, 208, 304) as a function of time during the measurement cycle;
- determining a velocity and/or an acceleration of the magnetic elongated probe, based on the sensor data; and
- determining a property of the object (204), based on a change in the velocity and/or the acceleration of the magnetic elongated probe.

19. A method according to any of claims 12-18 wherein the method further comprises comparing the measured first induced voltage as function of time with a second predetermined criterion, and if based on the comparison the second predetermined criterion is met, use it as an indication to provide a warning indicator on a user interface of the rebound tonometer (100, 202, 300).

20. A method according to claim 19, wherein the second predetermined criterion is selected to be one of: a second predetermined voltage threshold value, a second integral value of the measured first induced voltage as function of time over the first time period, a second derivate value of the first induced voltage as function of time or a second predetermined pattern.

## Patentansprüche

1. Rückpralltonometer (100, 202, 300), umfassend:
einen Körper (102, 206, 302), der ein proximales Ende (110, 214) und ein distales Ende (112, 216) aufweist, das dem proximalen Ende gegenüberliegt, wobei der Körper an dem proximalen Ende eine Öffnung (114) aufweist;
eine magnetische längliche Sonde (104, 208, 304), die ein erstes Ende (116, 218) und ein zweites Ende (118, 220) aufweist, das dem ersten Ende gegenüberliegt, wobei die magnetische längliche Sonde mindestens teilweise so in dem Körper angeordnet ist, dass das erste Ende aus der Öffnung des Körpers herausragt, das erste Ende in einem ersten Abstand (D1) von der Öffnung liegt und das zweite Ende im Inneren des Körpers liegt, und wobei die magnetische längliche Sonde angeordnet ist, um an einer Achse (120) des Rückpralltonometers ausgerichtet und entlang dieser bewegbar zu sein;
eine Messspule (106, 210, 306) und eine Antriebsspule (108, 212, 308), die im Inneren des Körpers angeordnet sind und um die magnetische längliche Sonde teilweise zu umgeben; und
eine Steuerung (310), die konfiguriert ist zum:
- Erkennen eines Kontakts zwischen einem Objekt (204) und dem ersten Ende, wenn das Rückpralltonometer in Verwendung ist, durch:
- Erregen der Antriebsspule, um die magnetische längliche Sonde in Bezug auf den Körper zu bewegen, um das erste Ende in einem zweiten Abstand (D2) von dem proximalen Ende aufzuweisen, wobei der zweite Abstand größer als der erste Abstand ist;
- Messen einer ersten induzierten Spannung in der Messspule in Abhängigkeit von einer Zeit während einer ersten Zeitspanne, und
- Vergleichen der gemessenen ersten induzierten Spannung in Abhängigkeit von der Zeit mit einem zuvor bestimmten Kriterium, und falls das zuvor bestimmte Kriterium basierend auf dem Vergleich erfüllt ist, Verwenden davon als ein Hinweis auf einen erkannten Kontakt zwischen dem ersten Ende und dem Objekt; und
- Einleiten eines Messzyklus des Rückpralltonometers, wenn der Kontakt erkannt wird.

2. Rückpralltonometer (100, 202, 300) nach Anspruch 1, wobei das zuvor bestimmte Kriterium ausgewählt ist, um eines zu sein von: einem zuvor bestimmten Spannungsschwellenwert, einem Integralwert der gemessenen ersten induzierten Spannung in Abhängigkeit von der Zeit über die erste Zeitspanne, einem Ableitungswert der ersten induzierten Spannung in Abhängigkeit von der Zeit oder einem zuvor bestimmten Muster.

3. Rückpralltonometer (100, 202, 300) nach Anspruch 1 oder 2, wobei, wenn der Messzyklus des Rückpralltonometers eingeleitet wird, die Steuerung (310) konfiguriert ist zum:
- Erregen der Messspule (106, 210, 306), um die magnetische längliche Sonde (104, 208, 304) in den Körper (102, 206, 302) zurückzuziehen, um das erste Ende (116, 218) in dem ersten Abstand (D1) von dem proximalen Ende (110, 214) aufzuweisen; und
- Erregen der Antriebsspule (108, 212, 308), um die magnetische längliche Sonde aus dem Körper auszustoßen, um das erste Ende in einem dritten Abstand (D3) von dem proximalen Ende aufzuweisen.

4. Rückpralltonometer (100, 202, 300) nach Anspruch 3, wobei der dritte Abstand in einem Bereich von einem 0,5- bis 2,0-Fachen des zweiten Abstands liegt.

5. Rückpralltonometer (100, 202, 300) nach Anspruch 3 oder 4, wobei, wenn der Abstand des Objekts (204) von dem proximalen Ende (110, 214) gleich dem zweiten Abstand ist, die Steuerung (310) ferner konfiguriert ist zum:
- Auswählen des dritten Abstands, um gleich oder größer als der zweite Abstand zu sein; und
- Abschließen des Messzyklus des Rückpralltonometers durch Messen einer zweiten Spannung, die in der Messspule (106, 210, 306) induziert wird, in Abhängigkeit von der Zeit während einer zweiten Zeitspanne des Ausstoßes der magnetischen länglichen Sonde (104, 208, 304), wobei die zweite Spannung auf eine Kollision des ersten Endes (116, 218) mit dem Objekt hinweist.

6. Rückpralltonometer (100, 202, 300) nach Anspruch 5, wobei, wenn der ausgewählte dritte Abstand größer als das 2-Fache des zweiten Abstands ist, die Steuerung (310) ferner konfiguriert ist, um auf einer Benutzerschnittstelle des Rückpralltonometers einen Hinweis bereitzustellen, um den Abstand zwischen dem Objekt (204) und dem proximalen Ende (110, 214) des Körpers (102, 206, 302) zu vergrößern.

7. Rückpralltonometer (100, 202, 300) nach Anspruch 3 oder 4, wobei, wenn der Abstand des Objekts (204) von dem proximalen Ende (110, 214) kleiner als der zweite Abstand ist, die Steuerung (310) ferner konfiguriert ist zum:
- Auswählen des dritten Abstands, um kleiner als der zweite Abstand zu sein; und
- Abschließen des Messzyklus des Rückpralltonometers durch Messen einer zweiten Spannung, die in der Messspule (106, 210, 306) induziert wird, in Abhängigkeit von der Zeit während einer zweiten Zeitspanne des Ausstoßes der magnetischen länglichen Sonde (104, 208, 304), wobei die zweite Spannung auf die Kollision des ersten Endes (116, 218) mit dem Objekt hinweist.

8. Rückpralltonometer (100, 202, 300) nach einem der Ansprüche 5 bis 7, wobei die Steuerung (310) ferner konfiguriert ist, um die zweite Spannung zu verwenden, um eine Eigenschaft des Objekts (204) zu bestimmen.

9. Rückpralltonometer (100, 202, 300) nach einem der vorstehenden Ansprüche, ferner umfassend mindestens einen Sensor (312), wobei die Steuerung (310) konfiguriert ist zum:
- Erfassen, von dem mindestens einen Sensor, von Sensordaten, die auf eine Verschiebung und/oder Geschwindigkeit der magnetischen länglichen Sonde (104, 208, 304) hinweisen, in Abhängigkeit von der Zeit während des Messzyklus;
- Bestimmen einer Geschwindigkeit und/oder einer Beschleunigung der magnetischen länglichen Sonde basierend auf den Sensordaten; und
- Bestimmen einer Eigenschaft des Objekts (204) basierend auf einer Änderung der Geschwindigkeit und/oder der Beschleunigung der magnetischen länglichen Sonde.

10. Rückpralltonometer (100, 202, 300) nach einem der vorstehenden Ansprüche, wobei die Steuerung (310) ferner konfiguriert ist, um die gemessene erste induzierte Spannung in Abhängigkeit von der Zeit mit einem zweiten zuvor bestimmten Kriterium zu vergleichen und, falls das zweite zuvor bestimmte Kriterium basierend auf dem Vergleich erfüllt ist, dies als einen Hinweis zu verwenden, um einen Warnhinweis auf einer Benutzerschnittstelle des Rückpralltonometers bereitzustellen.

11. Rückpralltonometer (100, 202, 300) nach Anspruch 10, wobei das zweite zuvor bestimmte Kriterium ausgewählt ist, um eines zu sein von: einem zweiten zuvor bestimmten Spannungsschwellenwert, einem zweiten Integralwert der gemessenen ersten induzierten Spannung in Abhängigkeit von der Zeit über die erste Zeitspanne, einem zweiten Ableitungswert der ersten induzierten Spannung in Abhängigkeit von der Zeit oder einem zweiten zuvor bestimmten Muster.

12. Verfahren zur Verwendung eines Rückpralltonometers (100, 202, 300) zum Bestimmen einer Eigenschaft eines Objekts (204), wobei das Rückpralltonometer einen Körper (102, 206, 302), eine magnetische längliche Sonde (104, 208, 304), eine Messspule (106, 210, 306) und eine Antriebsspule (108, 212, 308) umfasst, das Verfahren umfassend:
- Anordnen der magnetischen länglichen Sonde mindestens teilweise in dem Körper so, dass ein erstes Ende (116, 218) der magnetischen länglichen Sonde aus einer Öffnung (114) an einem proximalen Ende (110, 214) des Körpers aus dem Körper herausragt, das erste Ende in einem ersten Abstand von der Öffnung liegt und ein zweites Ende (118, 220) der magnetischen länglichen Sonde im Inneren des Körpers liegt, wobei die magnetische längliche Sonde angeordnet ist, um entlang einer Achse (120) des Rückpralltonometers bewegbar zu sein;
- Erkennen eines Kontakts zwischen dem Objekt und dem ersten Ende durch:
- Bewegen der magnetischen länglichen Sonde in Bezug auf den Körper, um das erste Ende in einem zweiten Abstand von dem proximalen Ende aufzuweisen, wobei der zweite Abstand größer als der erste Abstand ist;
- Bewegen des Rückpralltonometers in Bezug auf das Objekt während einer ersten Zeitspanne;
- Messen einer ersten induzierten Spannung in Abhängigkeit von der Zeit während einer ersten Zeitspanne, während das Rückpralltonometer bewegt wird, und
- Vergleichen der gemessenen ersten induzierten Spannung in Abhängigkeit von der Zeit mit einem zuvor bestimmten Kriterium, und falls das zuvor bestimmte Kriterium basierend auf dem Vergleich erfüllt ist, Verwenden davon als Hinweis auf einen erkannten Kontakt zwischen dem ersten Ende und dem Objekt;
- Einleiten eines Messzyklus des Rückpralltonometers, wenn der Kontakt erkannt wird.

13. Verfahren nach Anspruch 12, wobei das zuvor bestimmte Kriterium ausgewählt ist, um eines zu sein von: einem zuvor bestimmten Spannungsschwellenwert, einem Integralwert der gemessenen ersten induzierten Spannung in Abhängigkeit von der Zeit über die erste Zeitspanne, einem Ableitungswert der ersten induzierten Spannung in Abhängigkeit von der Zeit oder einem zuvor bestimmten Muster.

14. Verfahren nach Anspruch 12 oder 13, wobei der Messzyklus umfasst:
- Erregen einer Messspule (106, 210, 306), um die magnetische längliche Sonde (104, 208, 304) in den Körper (102, 206, 302) zurückzuziehen, um das erste Ende (116, 218) in dem ersten Abstand von dem proximalen Ende (110, 214) aufzuweisen;
- Erregen der Antriebsspule (108, 212, 308), um die magnetische längliche Sonde aus dem Körper auszustoßen, um das erste Ende in einem dritten Abstand von dem proximalen Ende aufzuweisen;
- Abschließen des Messzyklus durch Messen einer zweiten Spannung, die in der Messspule in Abhängigkeit von der Zeit während einer zweiten Zeitspanne des Ausstoßens der magnetischen länglichen Sonde induziert wird,
wobei die zweite Spannung anzeigt, dass das erste Ende mit dem Objekt (204) kollidiert; und
- Verwenden der zweiten Spannung zum Bestimmen der Eigenschaft des Objekts.

15. Verfahren nach Anspruch 14, wobei, wenn der Abstand des Objekts (204) von dem proximalen Ende (110, 214) gleich dem zweiten Abstand ist, das Verfahren ferner das Auswählen des dritten Abstands umfasst, um gleich oder größer als der zweite Abstand zu sein.

16. Verfahren nach Anspruch 15, wobei, wenn der dritte Abstand größer als das 2-Fache des zweiten Abstands ist, das Verfahren ferner das Bereitstellen eines Hinweises umfasst, um den Abstand zwischen dem Objekt (204) und dem proximalen Ende (110, 214) des Körpers (102, 206, 302) auf einer Benutzerschnittstelle des Rückpralltonometers (100, 202, 300) zu vergrößern.

17. Verfahren nach Anspruch 14, wobei, wenn der Abstand des Objekts (204) von dem proximalen Ende (110, 214) kleiner als der zweite Abstand ist, das Verfahren ferner das Auswählen des dritten Abstands umfasst, um kleiner als der zweite Abstand zu sein.

18. Verfahren nach einem der Ansprüche 12 bis 17, ferner umfassend:
- Erfassen, von mindestens einem Sensor (312) des Rückpralltonometers (100, 202, 300), von Sensordaten, die auf die Verschiebung und/oder Geschwindigkeit der magnetischen länglichen Sonde (104, 208, 304) hinweisen, in Abhängigkeit von der Zeit während des Messzyklus;
- Bestimmen einer Geschwindigkeit und/oder einer Beschleunigung der magnetischen länglichen Sonde basierend auf den Sensordaten; und
- Bestimmen einer Eigenschaft des Objekts (204) basierend auf einer Änderung der Geschwindigkeit und/oder der Beschleunigung der magnetischen länglichen Sonde.

19. Verfahren nach einem der Ansprüche 12 bis 18, wobei das Verfahren ferner das Vergleichen der gemessenen ersten induzierten Spannung in Abhängigkeit von der Zeit mit einem zweiten zuvor bestimmten Kriterium umfasst, und falls das zweite zuvor bestimmte Kriterium basierend auf dem Vergleich erfüllt ist, dies als Hinweis darauf verwendet wird, einen Warnhinweis auf einer Benutzerschnittstelle des Rückpralltonometers (100, 202, 300) bereitzustellen.

20. Verfahren nach Anspruch 19, wobei das zweite zuvor bestimmte Kriterium ausgewählt ist, um eines zu sein von: einem zweiten zuvor bestimmten Spannungsschwellenwert, einem zweiten Integralwert der gemessenen ersten induzierten Spannung in Abhängigkeit von der Zeit über die erste Zeitspanne, einem zweiten Ableitungswert der ersten induzierten Spannung in Abhängigkeit von der Zeit oder einem zweiten zuvor bestimmten Muster.

## Revendications

1. Tonomètre à rebond (100, 202, 300) comprenant :
un corps (102, 206, 302) ayant une extrémité proximale (110, 214) et une extrémité distale (112, 216) opposée à l'extrémité proximale, dans lequel le corps a une ouverture (114) au niveau de l'extrémité proximale ;
une sonde magnétique allongée (104, 208, 304) ayant une première extrémité (116, 218) et une seconde extrémité (118, 220) opposée à la première extrémité, dans lequel la sonde magnétique allongée est disposée au moins partiellement dans le corps de manière à ce que la première extrémité fasse saillie à l'extérieur du corps à partir de l'ouverture du corps, que la première extrémité se trouve à une première distance (D1) de l'ouverture et que la seconde extrémité se trouve à l'intérieur du corps, et dans lequel la sonde magnétique allongée est disposée pour être alignée sur un axe (120) du tonomètre à effet de rebond et mobile le long de celui-ci ;
une bobine de mesure (106, 210, 306) et une bobine d'entraînement (108, 212, 308) disposées à l'intérieur du corps et entourant partiellement la sonde magnétique allongée ; et
un dispositif de commande (310) configuré pour :
- détecter un contact entre un objet (204) et la première extrémité, lorsque le tonomètre à rebond est en cours d'utilisation, par :
- l'alimentation de la bobine d'entraînement pour déplacer la sonde magnétique allongée par rapport au corps afin que la première extrémité se trouve à une deuxième distance (D2) de l'extrémité proximale, la deuxième distance étant supérieure à la première distance ;
- la mesure d'une première tension induite dans la bobine de mesure en fonction du temps pendant une première période de temps, et
- la comparaison de la première tension induite mesurée en fonction du temps à un critère prédéterminé et, si, sur la base de la comparaison, le critère prédéterminé est satisfait, l'utiliser comme une indication d'un contact détecté entre la première extrémité et l'objet ; et
- le lancement d'un cycle de mesure du tonomètre à rebond lorsque le contact est détecté.

2. Tonomètre à rebond (100, 202, 300) selon la revendication 1, dans lequel le critère prédéterminé est choisi pour être l'un parmi : une valeur seuil de tension prédéterminée, une valeur intégrale de la première tension induite mesurée
en fonction du temps sur la première période de temps, une valeur dérivée de la première tension induite en fonction du temps ou d'un modèle prédéterminé.

3. Tonomètre à rebond (100, 202, 300) selon la revendication 1 ou 2, dans lequel, lors du lancement du cycle de mesure du tonomètre à rebond, le dispositif de commande (310) est configuré pour :
- alimenter la bobine de mesure (106, 210, 306) pour rétracter la sonde magnétique allongée (104, 208, 304) dans le corps (102, 206, 302) afin que la première extrémité (116, 218) se trouve à la première distance (D1) de l'extrémité proximale (110, 214) ; et
- alimenter la bobine d'entraînement (108, 212, 308) pour éjecter la sonde magnétique allongée hors du corps afin que la première extrémité se trouve à une troisième distance (D3) de l'extrémité proximale.

4. Tonomètre à rebond (100, 202, 300) selon la revendication 3, dans lequel la troisième distance est comprise entre 0,5 et 2,0 fois la deuxième distance.

5. Tonomètre à rebond (100, 202, 300) selon la revendication 3 ou 4, dans lequel, lorsque la distance de l'objet (204) par rapport à l'extrémité proximale (110, 214) est égale à la deuxième distance, le dispositif de commande (310) est en outre configuré pour :
- sélectionner la troisième distance pour qu'elle soit égale ou supérieure à la deuxième distance ; et
- achever le cycle de mesure du tonomètre à rebond en mesurant une seconde tension qui est induite dans la bobine de mesure (106, 210, 306) en fonction du temps pendant une seconde période de temps de l'éjection de la sonde magnétique allongée (104, 208, 304), dans lequel la seconde tension indique une collision de la première extrémité (116, 218) avec l'objet.

6. Tonomètre à rebond (100, 202, 300) selon la revendication 5, dans lequel, lorsque la troisième distance sélectionnée est supérieure à deux fois la deuxième distance, le dispositif de commande (310) est en outre configuré pour fournir une indication pour augmenter la distance entre l'objet (204) et l'extrémité proximale (110, 214) du corps (102, 206, 302) sur une interface utilisateur du tonomètre à rebond.

7. Tonomètre à rebond (100, 202, 300) selon la revendication 3 ou 4, dans lequel, lorsque la distance de l'objet (204) par rapport à l'extrémité proximale (110, 214) est inférieure à la deuxième distance, le dispositif de commande (310) est en outre configuré pour :
- sélectionner la troisième distance pour qu'elle soit inférieure à la deuxième distance ; et
- achever le cycle de mesure du tonomètre à rebond en mesurant une seconde tension qui est induite dans la bobine de mesure (106, 210, 306) en fonction du temps pendant une seconde période de temps de l'éjection de la sonde magnétique allongée (104, 208, 304), dans lequel la seconde tension indique une collision de la première extrémité (116, 218) avec l'objet.

8. Tonomètre à rebond (100, 202, 300) selon l'une quelconque des revendications 5 à 7, dans lequel le dispositif de commande (310) est en outre configuré pour utiliser la seconde tension pour déterminer une propriété de l'objet (204).

9. Tonomètre à rebond (100, 202, 300) selon l'une quelconque des revendications précédentes, comprenant en outre au moins un capteur (312), dans lequel le dispositif de commande (310) est configuré pour :
- collecter, à partir de l'au moins un capteur, des données de capteur indiquant un déplacement et/ou une vitesse de la sonde magnétique allongée (104, 208, 304) en fonction du temps pendant le cycle de mesure ;
- déterminer une vitesse et/ou une accélération de la sonde magnétique allongée, sur la base des données de capteur ; et
- déterminer une propriété de l'objet (204), sur la base d'un changement de la vitesse et/ou de l'accélération de la sonde magnétique allongée.

10. Tonomètre à rebond (100, 202, 300) selon l'une quelconque des revendications précédentes, dans lequel le dispositif de commande (310) est en outre configuré pour comparer la première tension induite mesurée en fonction du temps avec un second critère prédéterminé, et si, sur la base de la comparaison, le second critère prédéterminé est satisfait, l'utiliser comme indication pour fournir un indicateur d'avertissement sur une interface utilisateur du tonomètre à rebond.

11. Tonomètre à rebond (100, 202, 300) selon la revendication 10, dans lequel le second critère prédéterminé est choisi pour être l'un parmi : une seconde valeur seuil de tension prédéterminée, une seconde valeur intégrale de la première tension induite mesurée en fonction du temps sur la première période de temps, une seconde valeur dérivée de la première tension induite en fonction du temps ou un second modèle prédéterminé.

12. Procédé d'utilisation d'un tonomètre à rebond (100, 202, 300) permettant de déterminer une propriété d'un objet (204), dans lequel le tonomètre à rebond comprend un corps (102, 206, 302), une sonde magnétique allongée (104, 208, 304), une bobine de mesure (106, 210, 306) et une bobine d'entraînement (108, 212, 308), le procédé comprenant :
- la disposition de la sonde magnétique allongée au moins partiellement dans le corps de manière à ce qu'une première extrémité (116, 218) de la sonde magnétique allongée fasse saillie à l'extérieur du corps à partir d'une ouverture (114) au niveau d'une extrémité proximale (110, 214) du corps, que la première extrémité se trouve à une première distance de l'ouverture, et qu'une seconde extrémité (118, 220) de la sonde magnétique allongée se trouve à l'intérieur du corps, dans lequel la sonde magnétique allongée est disposée pour être mobile le long d'un axe (120) du tonomètre à rebond ;
- la détection d'un contact entre l'objet et la première extrémité par :
- le déplacement de la sonde magnétique allongée par rapport au corps afin que la première extrémité se trouve à une deuxième distance de l'extrémité proximale, la deuxième distance étant supérieure à la première distance ;
- le déplacement du tonomètre à rebond par rapport à l'objet pendant une première période de temps ;
- la mesure d'une première tension induite en fonction du temps pendant une première période de temps lors du déplacement du tonomètre à rebond, et
- la comparaison de la première tension induite mesurée en fonction du temps à un critère prédéterminé et, si, sur la base de la comparaison, le critère prédéterminé est satisfait, l'utiliser comme indication d'un contact détecté entre la première extrémité et l'objet ;
- le lancement d'un cycle de mesure du tonomètre à rebond lorsque le contact est détecté.

13. Procédé selon la revendication 12, dans lequel le critère prédéterminé est choisi pour être l'un parmi : une valeur seuil de tension prédéterminée, une valeur intégrale de la première tension induite mesurée en fonction du temps sur la première période de temps, une valeur dérivée de la première tension induite en fonction du temps ou un modèle prédéterminé.

14. Procédé selon la revendication 12 ou 13, dans lequel le cycle de mesure comprend :
- l'alimentation d'une bobine de mesure (106, 210, 306) pour rétracter la sonde magnétique allongée (104, 208, 304) dans le corps (102, 206, 302) afin que la première extrémité (116, 218) se trouve à la première distance de l'extrémité proximale (110, 214) ;
- l'alimentation de la bobine d'entraînement (108, 212, 308) pour éjecter la sonde magnétique allongée hors du corps afin que la première extrémité se trouve à une troisième distance de l'extrémité proximale ;
- l'achèvement du cycle de mesure en mesurant une seconde tension qui est induite dans la bobine de mesure en fonction du temps pendant une seconde période de temps de l'éjection de la sonde magnétique allongée,
dans lequel la seconde tension indique que la première extrémité entre en collision avec l'objet (204) ; et
- l'utilisation de la seconde tension pour déterminer la propriété de l'objet.

15. Procédé selon la revendication 14, dans lequel, lorsque la distance de l'objet (204) par rapport à l'extrémité proximale (110, 214) est égale à la deuxième distance, le procédé comprend en outre la sélection de la troisième distance pour qu'elle soit égale ou supérieure à la deuxième distance.

16. Procédé selon la revendication 15, dans lequel lorsque la troisième distance est supérieure à deux fois la deuxième distance, le procédé comprend en outre la fourniture d'une indication pour augmenter la distance entre l'objet (204) et l'extrémité proximale (110, 214) du corps (102, 206, 302) sur une interface utilisateur du tonomètre à rebond (100, 202, 300).

17. Procédé selon la revendication 14, dans lequel, lorsque la distance de l'objet (204) par rapport à l'extrémité proximale (110, 214) est inférieure à la deuxième distance, le procédé comprend en outre la sélection de la troisième distance pour qu'elle soit inférieure à la deuxième distance.

18. Procédé selon l'une quelconque des revendications 12 à 17, comprenant en outre :
- la collecte, à partir d'au moins un capteur (312) du tonomètre à rebond (100, 202, 300), de données de capteur indiquant un déplacement et/ou une vitesse de la sonde magnétique allongée (104, 208, 304) en fonction du temps pendant le cycle de mesure ;
- la détermination d'une vitesse et/ou d'une accélération de la sonde magnétique allongée, sur la base des données de capteur ; et
- la détermination d'une propriété de l'objet (204), sur la base d'un changement de la vitesse et/ou de l'accélération de la sonde magnétique allongée.

19. Procédé selon l'une quelconque des revendications 12 à 18, dans lequel le procédé comprend en outre la comparaison de la première tension induite mesurée en fonction du temps à un second critère prédéterminé, et si, sur la base de la comparaison, le second critère prédéterminé est satisfait, l'utiliser comme une indication pour fournir un indicateur d'avertissement sur une interface utilisateur du tonomètre à rebond (100, 202, 300).

20. Procédé selon la revendication 19, dans lequel le second critère prédéterminé est choisi pour être l'un parmi : une seconde valeur seuil de tension prédéterminée, une seconde valeur intégrale de la première tension induite mesurée en fonction du temps sur la première période de temps, une seconde valeur dérivée de la première tension induite en fonction du temps ou un second modèle prédéterminé.
